# EUROPEAN PATENT APPLICATION

(11) **EP 2 184 367 A1**
(43) Date of publication of application: **12.05.2010**
(21) Application number: 08019150.5
(22) Date of filing: 31.10.2008
(51) Int. Cl.: C12Q 1/04, C12Q 1/10

(54) **Non-hydrolytic microbial probes**

(71) Applicant: Biosynth AG, 9422 Staad (CH)
(72) Inventor: Wick, Lukas, 8400 Winterthur (CH); Mayer, Thomas, 78351 Bodmnan-Ludwigshafen (DE); Spitz, Urs, Dr., 8704 Herrliberg (CH)
(74) Representative: Schmauder & Partner AG Patent- & Markenanwälte VSP

(57) **Abstract**

A method of detecting the presence of selected living cells in a sample, comprises the steps of contacting the sample with a substrate compound and monitoring for a signal caused by a metabolic activity of said selected living cells resulting in a release of a signalogenic moiety from the substrate compound. The substrate compound has the formula 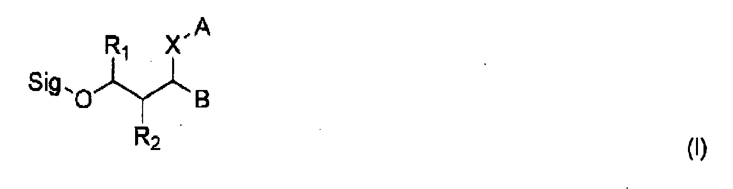 wherein X is O or NH, A is H or a first enzymatically hydrolyzable group, B is R₃ or CH-Y-D, wherein Y is O or NH and D is H or a second enzymatically hydrolyzable group, R₁, R₂ and R₃ are independently selected inert groups, and Sig-O is a signalogenic moiety that upon release thereof converts to said signalophore.

## Description

### Field of the invention

The present invention generally relates to the detection of selected living cells, particularly microbial species, by means of a novel class of hydrolytically stable substrates that allow the integration of assay and microbial sample enrichment.

### Background of the invention

Annually, there are more than 100 million assays performed in the United States alone to test for Salmonella or other food pathogens (see for example: Manafi M. New developments in chromogenic and fluorogenic culture media. Int J Food Microbiol. 2000, 60, 205-18).

Food, water and environmental samples often are very dilute and the sample volumes contain very small numbers of target organisms, the presence of which is often disguised by an overwhelming flora of ubiquitous organisms. It is well known that small populations of microbial contaminants in food products can rapidly multiply during handling, processing, storing or preparation. Such events are frequent causes of mass food poisoning as is evident by regular media coverage of such events.

Microbial testing has undergone massive development with the establishment of molecular (PCR) and immunological (ELISA) methods of assay. Compared to traditional culture, the novel methods are much faster. Results are often available within minutes or hours rather than days. However, such advantage manifests only if the to-be-assayed microbial organism is present at a rather high concentration. Dilute or minority populations often escape even the most advanced methods of detection. Therefore, assaying of dilute samples must involve an enrichment step, where the sample is brought into a liquid culture medium and incubated at conditions which are optimal for a target organism and allow it to outgrow any accompanying microbial flora.

The requirement for time consuming enrichment virtually eliminates all the time savings of expensive advanced assay techniques. Enrichment media remain indispensable and represent a significant component of the $1.5 billion global food testing market. Therefore, a successful integration of enrichment and assay may provide major cost and time savings over the state of the art.

Integration of enrichment and assay has been attempted numerous times by the addition of signalogenic microbial substrates (i.e. substrates that release a detectable signal upon microbial metabolization) to the enrichment medium in order to detect pathogens, for instance by indicating the activity of a certain biomarker enzyme (see for example: Lawrence R. et al. Isolation and detection of Listeria monocytogenes using fluorogenic and chromogenic substrates for phosphatidylinositol-specific phospholipase C. J. Food Prot. 1999, 62, 244-251).

In practice the approach has not been successful for two main reasons.

Firstly, common signalogenic microbial substrates typically are esters or glycosides of phenolics and are not fully stable under the conditions of enrichment. This lack of stability in aqueous environments manifests by a degree of non-specific hydrolysis. Non-specific hydrolysis is unrelated to the presence of microbial activity and thus creates undesirable background signal which often confuses the assay and causes false positive results.

Secondly, common signalogenic microbial substrates are not very specific indicators for target pathogens. Often these substrates are indicators for common hydrolases of limited use as biomarkers. This lack of specificity of microbial substrates further adds to the high number of false positive results.

### Summary of the Invention

According to the present invention, there is provided a method of detecting the presence of selected living cells in a sample, comprising the steps of:
a) contacting the sample with a compound having the formula: 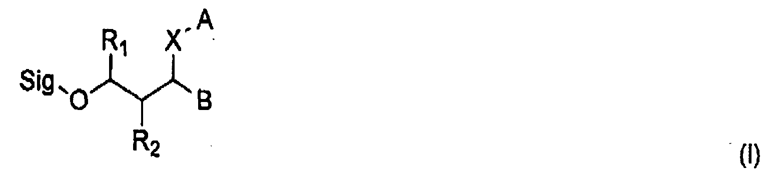 wherein:
   X is O or NH,
   A is H or a first enzymatically hydrolyzable group,
   B is R₃ or CH-Y-D, wherein Y is O or NH and D is H or a second enzymatically hydrolyzable group,
   T₁, R₂ and R₃ are independently selected inert groups, and
   Sig-O is a signalogenic moiety that upon release thereof converts to a signalophore, and
b) monitoring for a signal from said signalophore caused by a metabolic activity of said selected living cells, said activity resulting in a release of said signalogenic moiety.

As seen from structural formula (I), the substrates of this invention contain the structural element of a 1,3-dihydroxypropane (in the case of X = O) or a 1-hydroxy-3, aminopropane (in the case of X = NH) wherein the hydrogen of the 1-hydroxyl group has been replaced by a substituent Sig. The hydrogen of the 3-hydroxyl group or a hydrogen of the 3-amino group is optionally replaced by a first enzymatically hydrolyzable group. Moreover, the 3-carbon atom carries a substituent that is either an inert group or a group as defined above, which may include a second enzymatically hydrolyzable group.

The substituent Sig is chosen so that Sig-O functions as a signalogenic moiety: if the fragment Sig-O is a liberated by cleavage of the O-C1 bond in Compound (I), it will convert to a signalophore, i.e. to a species that can provide a detectable signal.

The signal may be of transient or persisting nature, and it may occur spontaneously (e.g. chemiluminescence or color change) or might require appropriate interrogation means such as optical excitation (e.g. fluorescence).

The Compounds (I) can be used for the detection of selected living cells which have the ability (alone or in concert) to oxidize primary and secondary aliphatic alcohols, vicinal diols, or vicinal amino alcohols that are derived of 1,3-dihydroxypropane or 1-hydroxy-3-aminopropane. The reliance on this non-hydrolytic metabolic activity forms the basis of the substrates of this invention, which are hydrolytically stable under the conditions of incubation and enrichment. In other words, the mere presence of a hydrolytic enzymatic activity is not sufficient to release the signalophore and generate a signal. The substrates are tunable to achieve high degrees of microbial specificity. This provides potentially significant advantages with respect to simplicity and time efficiency over presently known ways of microbial assaying.

It should be noted that according to the method of this invention the liberation of the Sig-O group and consequent generation of a detectable signal does not require the addition of an oxidant such as periodate.

Advantageous embodiments of the invention are defined in the dependent claims.

In particular, the enzymatically hydrolyzable groups are independently selected from a group that is labile towards the action of:
- **glycosidases,** including alpha-amylases, alpha-D-arabinosidases, alpha-L-arabinosidases, beta-D-cellobiosidases, alpha-D-fucosidases alpha-L-fucosidases, beta-D-fucosidases, beta-L-fucosidases, alpha- galactosaminidases, beta-galactosaminidases, alpha-galactosidases, beta-galactosidases, alpha-glucosaminidases, beta-glucosaminidases, alpha-glucosidases, beta-glucosidases, beta-glucuronidases, beta-lactosidases, alpha-maltosidases, beta-maltosidases, alpha-mannosidases, beta-mannosidases, neuraminidases, alpha-rhamnosidases, alpha-xylosidases, beta-xylosidases, as well as enzymes such alpha-L-arabinofiuranosideases, beta-chitobiosidases, galactopyranoside-6-sulfatases and beta-D-ribofuranosidases,
- **peptidases,** including L-alanine aminopeptidases, aminopeptidases A, aminopeptidases B, aminopeptidases M, dipeptidyl-aminopeptidases I, dipeptidyl-aminopeptidases II, dipeptidyl-aminopeptidases III, dipeptidyl-aminopeptidases IV, gamma-glutamyl transferases, hippurases, L-proline aminopeptidases, proline arylamidases, prolyl endopeptidases, proglutamyl peptidases I, and
- **ureidases,**
- **esterases,** including carboxylesterases,
- **lipases,** including cholinesterase,
- **phosphatases,** including alkaline phosphatases, acidic phosphatases, phosphodiesterases, endo and exo, pyrophosphatases, DNAses and ATPases,
- **sulfatases,** including arylsulfatases and glycosulfatases,
- **phospholipases A, B and C,** including inositol and choline specific phospholipases C,
- **dealkylases,** or
- **nitroreductases.**

### Detailed description of the Invention

The present invention is based on the discovery that distinct groups of microbial species, particularly but not only bacteria, have the ability to metabolize certain derivatives of aliphatic alcohols, vicinal diols and vicinal amino alcohols. This process is accompanied by the release of a detectable metabolite. In a preferred embodiment, detection of said metabolite occurs readily by measurement of change in optical absorption, emission, electric current or potential or simply by visual inspection.

Specifically, it was discovered that microbial metabolization of 1-aryloxy-3-propanols is associated with the release of phenols (ArOH). This process must involve the cleavage of a thermodynamically favorable C-O bond that, however, is well known to be inert towards hydrolysis under the conditions of incubation or enrichment.

Moreover, it was discovered that the aryloxy group of 1-aryloxy-3-propanol is well suited to act as a signalogen (e.g. a fluorogen, chromogen, luminogen or electrogen) to be released upon incubation (and thereby providing a detectable signal) with a wide range of microbial species thereby manifesting microbial activity.

For example, it was observed that 1-(4-nitrophenyloxy)-3-propanol (Table 1, Substrate 1) is degraded by a very narrow group of bacteria which most prominently includes the notorious infant formula pathogen *E. sakazakii.* The process of degradation which is highly indicative of presence and growth of *E. sakazakii* can readily be followed by the appearance of the yellow 4-nitrophenol absorption during culturing of a sample in the presence 1-(4-nitrophenyloxy)-3-propanol using a standard commercial plate reader.

The specificity of the substrate for *E*. *sakazakii* strongly relates to its chemical structure: Removal or replacement of the hydroxyl group such as in Substrates **2, 3** or **4** (Table 1) eliminates susceptibility towards *E. sakazakii* completely (Table 2). Interestingly, specificity of the substrate is maintained and the rate of substrate turnover (sensitivity) increased if the propane chain is elongated by one carbon atom such as in Substrate **5.**

White such discovery is certainly useful considering the need for safe dairy products, a more generally applicable platform for microbial pathogen assay is also desirable.

Surprisingly, it was found that specific chemical modification of the 1-aryloxy-3-propanol structure can alter substrate susceptibility towards certain microbial organisms completely.

For instance, the formal addition of a carbinol (-CH₂OH) group to the C3 position of the 1-aryloxy-3-propanol structure leads to 1-aryloxy-3,4-dihydroxybutane, which is much more susceptible to microbial degradation.

For example, it was found that 1-(coumarin-7-oxy)-3,4-dihydroxybutane (Table 1, Substrate **6**) is a useful indicator for *Enterobacteriaceae* by virtue of the appearance of 7-hydroxycoumarin fluorescence during incubation (Table 2).

Substrate **6** was found to be susceptible to *Citrobacter, Enterobacter, Hafina, Klebsiella, Escherichia, Morganella, Proteus, Serratia (except for S. rubidea), Salmonella and Shigella spp*. but not to *Yersinia and Providencia spp*. Outside the family of *Enterobacteriaceae*, only *Bacillus* and some *Aeromonas* species gave positive results (Table 2).

Without being bound by theory, this greatly expanded susceptibility appears to be due to the additional hydroxyl functionality in Substrate **6:** The above mentioned Substrate **5,** for example, closely resembles Substrate **6** but is lacking the second hydroxyl group. Substrate **5,** much like Substrate **1,** is susceptible to a very narrow band of species only.

The role of the second hydroxyl group is further evidenced by the fact that Substrates **7, 8, 9** and **10** (Table 1) where the hydroxyl replacement group is replaced by bromo (Substrate **7**), chloro (Substrate **8**), azido (Substrate **9**) and cyano (Substrate **10**) substituents, respectively, mostly lack the desired microbial susceptibility (Table 2).

Most interestingly, if the second hydroxyl group is replaced by an amino group such as in Substrate **11** (Table 1), the substrate retains susceptibility to a wide range of *Enterobacteriacae*. This range however, is narrower than the range covered by Substrate **6:** Substrate **11** can be used for assay of *Citrobacter, Escherichia* and *Salmonella* but is inert towards *Enterobacter, Nafina, Klebsiella, Morganella, Proteus* and *Shigella spp*., which can readily be assayed with Substrate **6** (Table 2).

Naturally, the question arises how these microbial substrates work. Without being bound by theory, it appears that the substrates are degraded by certain metabolic enzymes which represent specific features of certain bacterial species, genii or families. Such enzymes thus could be considered biomarkers for activity and growth of the corresponding organisms. Stated differently, the substrates can be used for the detection of metabolic states of living cells.

The requirement for a 3-hydroxyl group in the side chain of a phenolic ether points to a group of enzymes such as dehydrogenases with the ability to oxidize such hydroxyl group yielding a carbonyl functionality. Carbonyl compounds of this nature are well known to undergo *beta*-elimination which, applied to the present situation, results in the release of the phenol (ArOH) as observed by experiment (Scheme 1). See for example: Klein G. et al. An Enantioselective Fluorometric Assay for Alcohol Dehydrogenases Using Albumin-Catalyzed beta-Elimination of Umbelliferone. Bioorg Med Chem Letters. 1998;8:1113-6.

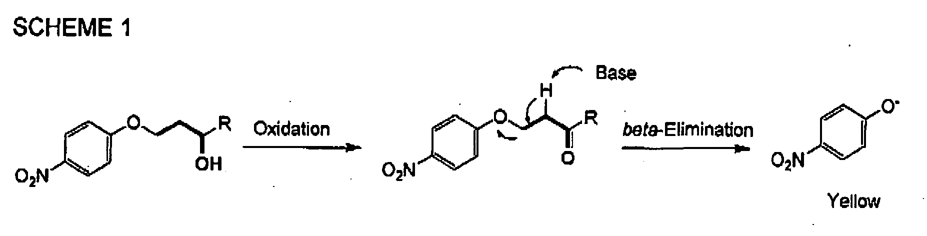

The postulated mechanism of action is in line with the observation that neither Substrates **12** nor **13** release a detectable chromogen upon incubation with any of the available test strains. These structures are readily oxidized but cannot undergo the postulated subsequent *beta*-eliminatian.

The much expanded susceptibility of the bi-functional vicinal diol or amino alcohol may well be explained by the wider range of microbial enzymes available to oxidize the substrates. Vicinal diols and amino alcohols are ubiquitous molecular motifs in nature (e.g. carbohydrates), hence it is plausible that a larger variety of corresponding metabolic enzymes exists. In other words, the presence of two hydroxyl groups in Substrate **6** may be seen as a logical "CJR" connection regarding susceptibility to non-hydrolytic enzymatic activity.

Enzymatic or chemical oxidation of a substrate which appears to play an important role in the functioning of disclosed microbial substrates can readily be prevented by masking the target functionality. For instance, the oxidation of an amine can be prevented by acylation. Similarly, the esterification of a hydroxyl group may serve to protect against oxidation. In addition, tertiary alcohols cannot readily be oxidized because this would require breakage a carbon-carbon bond.

This may explain why Substrate **14** (Table 1), where the hydroxyl group is tertiary and the amino group is acetylated, does not act as a substrate to any of the species tested (no release of a detectable fluorophore).

In contrast, Substrate **15,** where the hydroxyl group is masked by an ester and the amino group is acylated, represents a most useful, highly selective substrate that apparently is susceptible only to species of the genus Salmonella. Since Substrate **15** is inert even to chemical oxidation by periodate, (which in a different context serves perfectly well to titrate Substrates **6** and **11** - see for example: Badalassi F. et al. A Versatile Periodate-Coupled Fluorogenic Assay for Hydrolytic Enzymes. Angew Chem Int Ed Engl. 2000, 39, 4067-70), it appears unlikely that it can be oxidized by an enzyme in the manner postulated.

The structure of Substrate **15** is not the result of some random chemical synthesis. The structure was selected in view of the fact that Salmonella possesses significant C8-esterase activity in addition to L-alanine aminopetidase activity, which is indicative of gram negative species such as *Salmonella spp.*

Therefore, the observation that initially appeared to contradict the postulated enzymatic oxidation, in reality provides excellent support of the hypothesis: If Substrate **15** is subjected to hydrolysis by Salmonella esterases and peptidases, it is expected to yield Substrate **11** - which itself is an efficient fluorogenic substrate for *Enterobacteriacae* including *Salmonella spp*.

The above convincingly demonstrates that masking the functionalities of substrates such as Substrate **6** and Substrate 11 with enzyme labile groups provides a highly useful feature in that it allows to narrow down the microbial susceptibility of the substrate in a controlled way, making it possible to assay individual genii or species or possibly even strains.

Stated differently, these are signalogenic microbial substrates that yield a detectable signal only if the substrate is sequentially transformed by two or more enzymes, one of which possesses the ability to oxidize primary and secondary aliphatic alcohols, vicinal diols, or vicinal amino alcohols. In other words, these substrates may be seen as a logical "AND" connection regarding susceptibility to a non-hydrolytic and to a hydrolytic enzymatic activity.

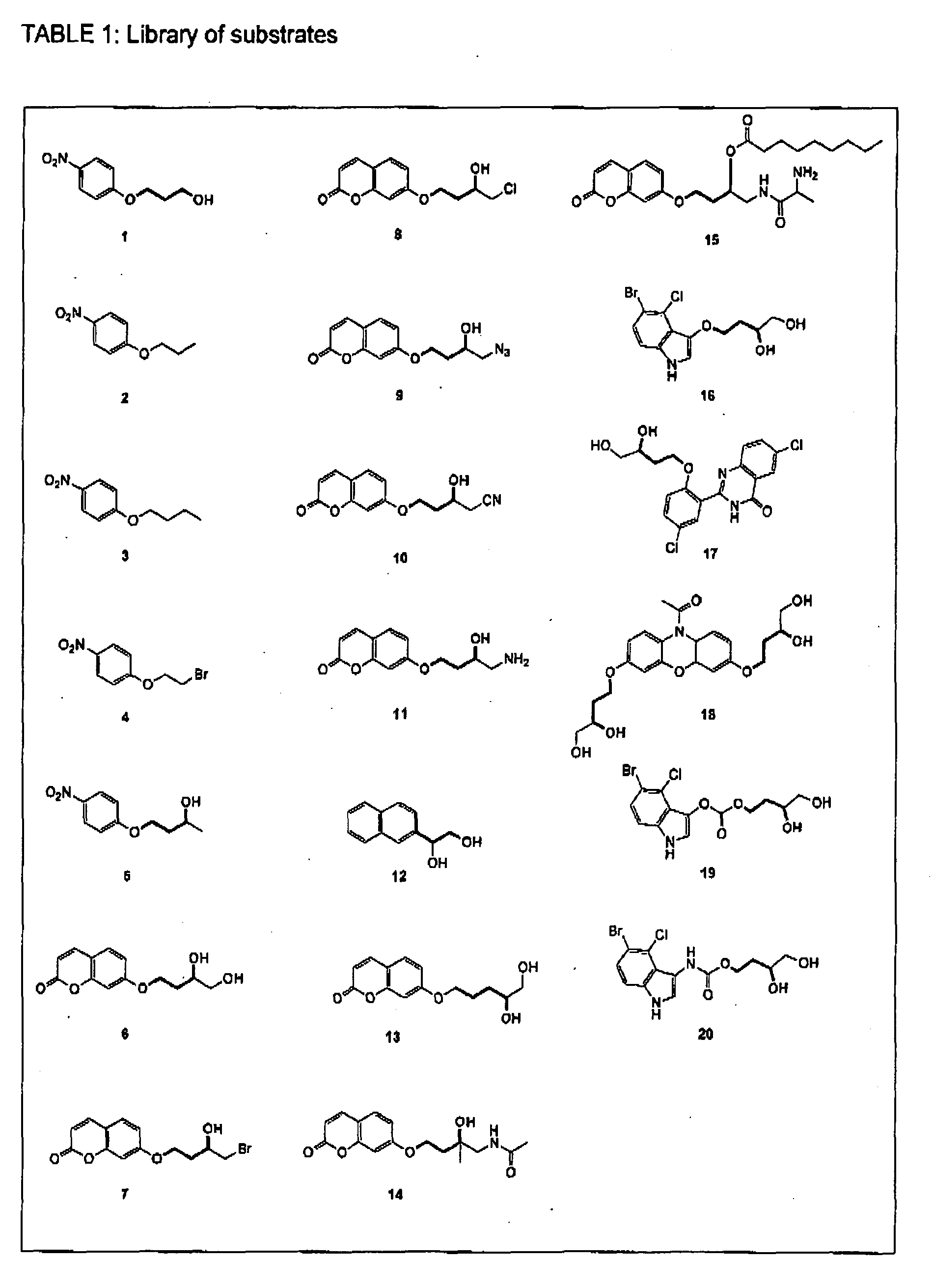

One might argue that such secondary enzyme labile groups simply probe for hydrolytic enzymes and hence defeat the purpose of providing non-hydrolytic substrates.

However, this is not the case because it is well known that the rate of non-specific hydrolysis of enzyme labile groups attached to *aliphatic* functionalities is several orders of magnitude lower as compared to their *aromatic* analogues (see for example: Badalassi F. et al. A Versatile Periodate-Coupled Fluorogenic Assay for Hydrolytic Enzymes. Angew Chem Int Ed Engl. 2000, 39, 4067-70).

While the examples presented here used 4-nitrophenol or 7-hydoxycoumarines, this is not to imply that the detectable signalophore ArOH is limited to 4-nitrophenol or 7-hydroxycoumarines.

In fact, a number of other substrates such as 3'-indoxyl-butan-1,2-diol (Substrate **16**) have been prepared and successfully tested, thereby establishing the general validity of the general concept presented here. For example, Substrate **16** upon microbial metabolization releases indoxyl, which is well known to form indigo by oxidative dimerization. The substrate has been successfully tested in microbial plating media for the staining of *Enterobacteriacae.*

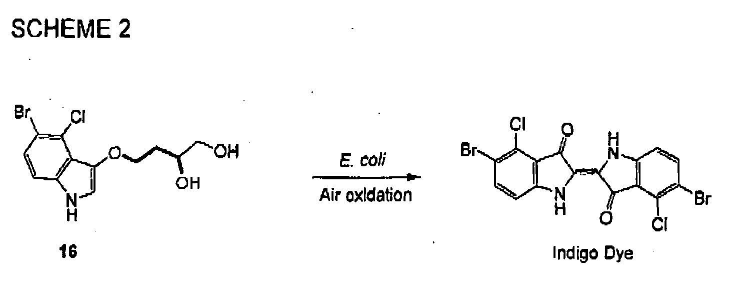

In order to test the general validity of the concept additional subtracted derived of various types of signalogens were tested including Substrate **17** (fluorogen: quinazoline family) and **18** (fluorogen / chromogen: phenoxazine family).

In principle, any type of signalogen can be employed in the context of the present invention. However, suitable signalogens (chromogens, fluorogens, luminogens) will typically be derived of the families of
■ azo-dyes (chromogenic)
■ acridanes (luminogenic)
■ benzenes (chromogenic)
■ benzimidazoles (fluorogenic)
■ benzothiazoles (fluorogenic)
■ benzoxazoles (fluorogenic)
■ bipyridyls (fluorogenic / chromogenic)
■ catechols (chromogenic metal chelators)
■ coumarines (fluorogenic)
■ dioxetanes (luminogenic)
■ flavones (chromogenic metal chelators)
■ lucifierins (luminogenic)
■ napthols (chromogenic)
■ nitrobenzenes (chromogenic, electrogenic)
■ indoles (chromogenic formation of indigo type dyes)
■ indolinones (fluorogenic)
■ quinazolines (fluorogenic)
■ quinolines (chromogenic and fluorogenic metal chelators)
■ phenoxazines (fluorogenic)
■ triphenylmethane dyes (chromogenic)

While testing various substrates it was observed that the postulated *beta*-elimination occurs at vastly different rates depending on the chemical nature of the substrate. In some cases non-fluorescent long-lived carbonyl intermediates are produced that cannot be readily detected.

Therefore, in a preferred embodiment, *beta*-elimination proceeds at a rapid rate under the conditions of the assay.
It is well known that *beta*-elimination is accelerated by certain proteins such as BSA, which in a preferred embodiment is added to the assay (see for example: Klein G. et al. An Enantioselective Fluorometric Assay for Alcohol Dehydrogenases Using Albumin-Catalyzed beta-Elimination of Umbelliferone. Bioorg Med Chem Letters. 1998; 8,1113-6).

According to another embodiment, the *beta*-elimination step can be accelerated by linking the signalogen and the hydroxypropane enzyme labile group in such way that *beta*-elimination and release of signalogen are accompanied by the extrusion of carbon dioxide, which provides a substantial thermodynamic driving force for the reaction. Examples are represented by Substrates **19** and **20** where the moiety X of Compound (I) is an aryloxycarbonyl rest (Scheme 3). Alternatively, moiety X can be an arylaminocarbonyl rest:

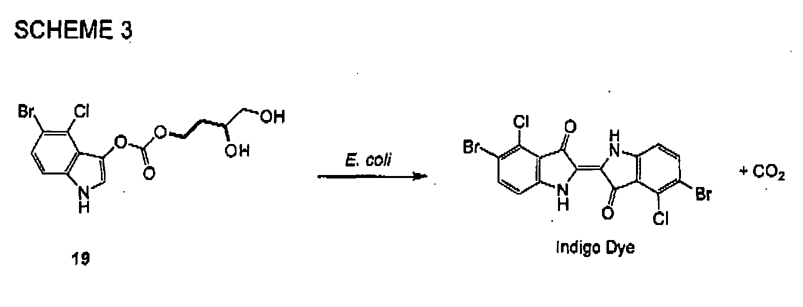

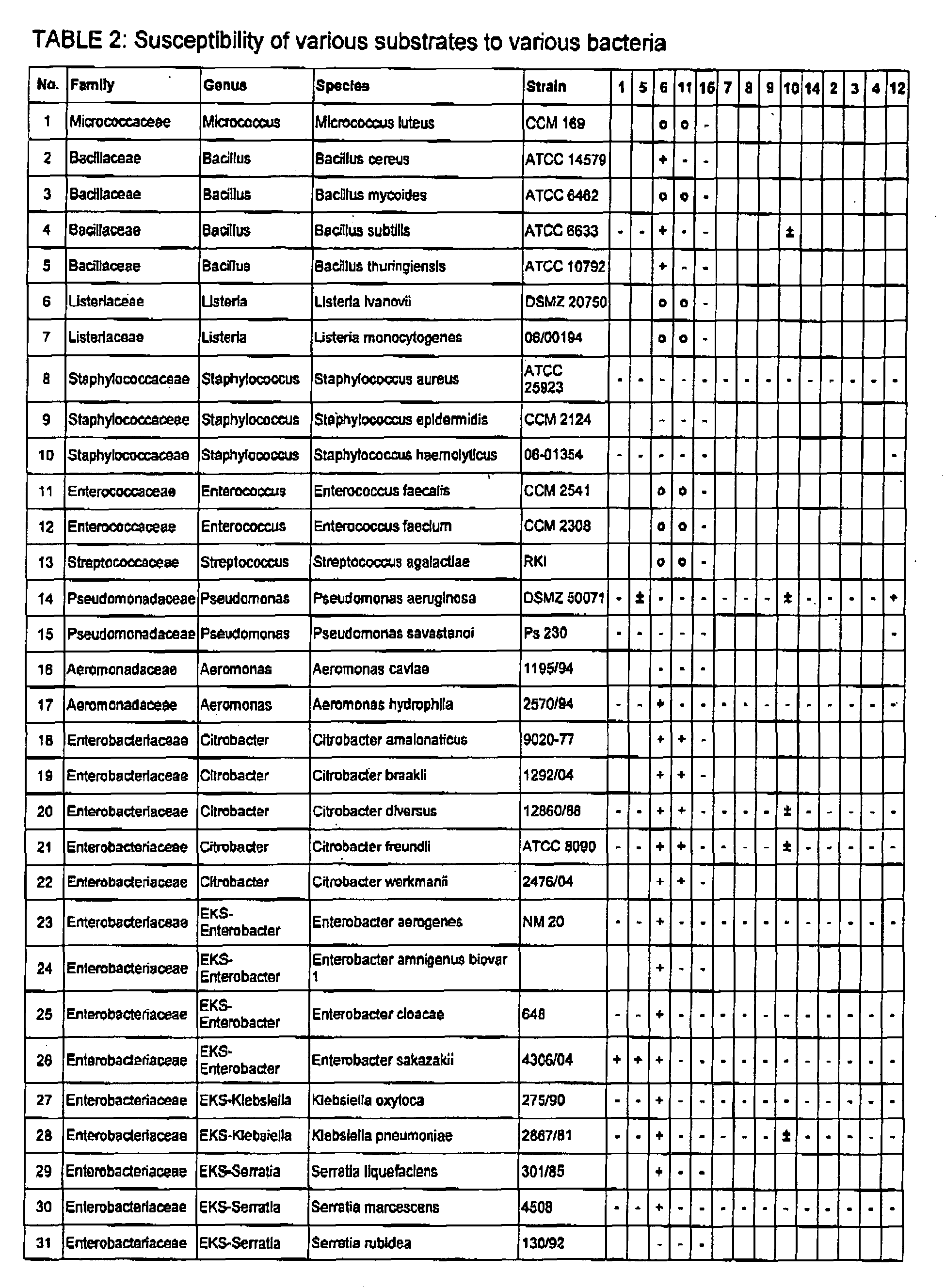

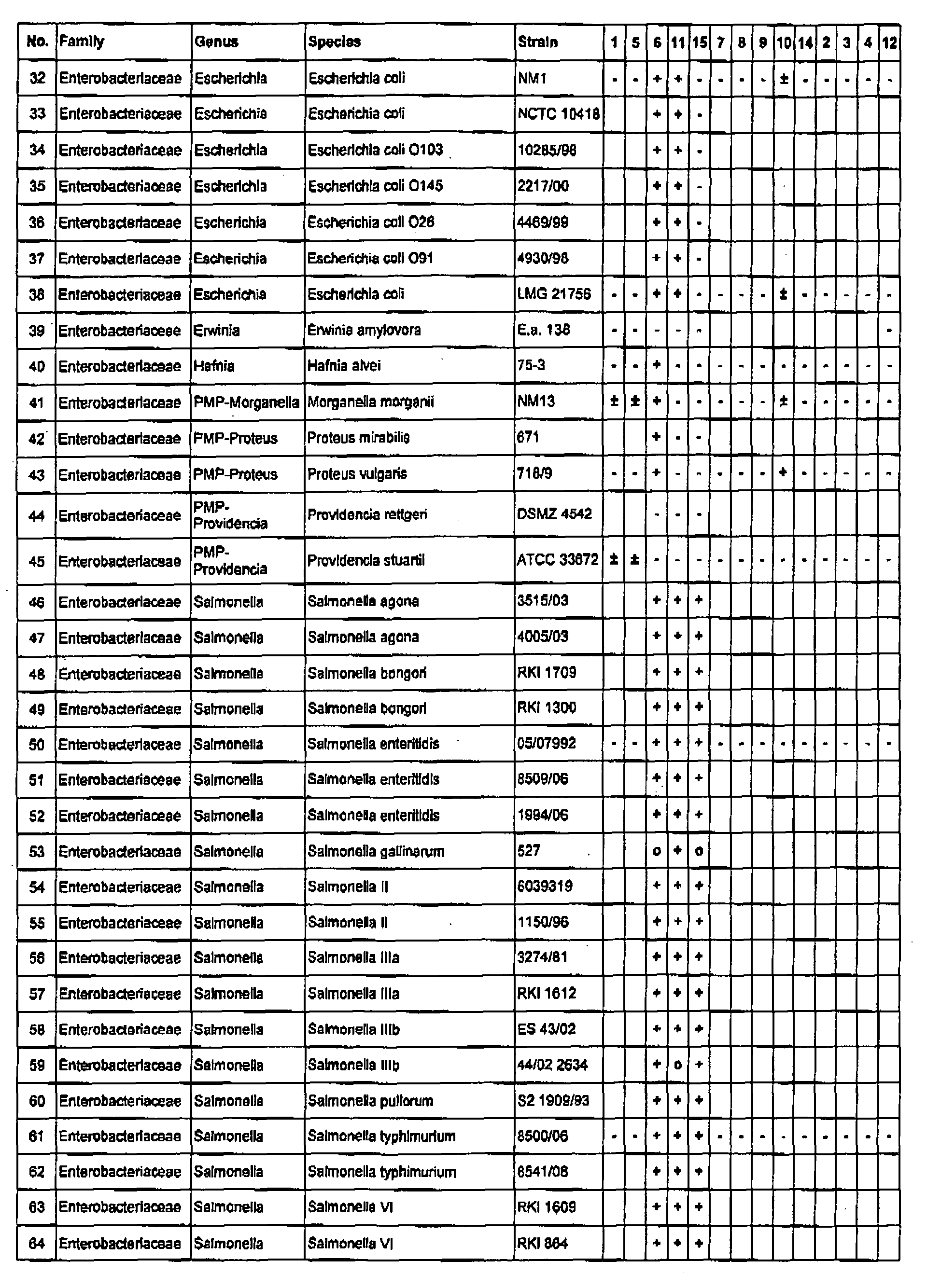

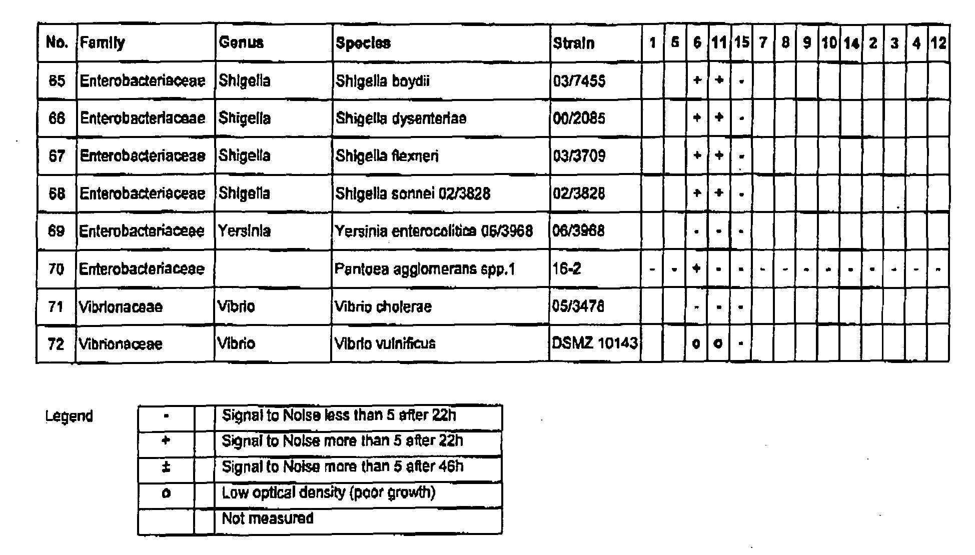

### EXAMPLES

Substrates **1** to **5, 7** to **10, 12** and **14** were obtained from Protéus SA, France. Substrates **6** and **11** were prepared according to methods published in the literature (see for example: Nyfeler E. et al. A Sensitive and Selective High-Throughput Screening Fluorescence Assay for Lipases and Esterases. Helv Chim Acta 2003;86:2919-27). The preparation of other substrates is disclosed further below.

### Example 1: Assay of E. sakazakii

Samples were inoculated in phosphate buffer pH 8 (with small amount of yeast extract and peptone) containing acetonitrile (control) or Substrates **1** or **5** in 2 mM concentration and incubated for 22 hours at 37°C. Relative absorbance (Abs420nm / Abs600nm) was measured using a standard plate reader. Results are shown in Table 3.

**TABLE 3: Assay of E. sakazakii with Substrates 1 and 5 versus acetonitrile control (C).**

| **Family** | **Genus** | **Species** | **Strain** | **C** | **1** | **5** |
|---|---|---|---|---|---|---|
| Aeromonadaceae | Aeromonas | Aeromonas hydrophila | 2570/94 | 1.5 | 1.8 | 1.5 |
| Bacillaceas | Bacillus | Bacillus subtilis | ATCC 6633 | 1.7 | 1.7 | 1.7 |
| Enterobacteriaceae | Citrobacter | Citrobacter diversus | 12860/88 | 1.5 | 2.0 | 1.7 |
| Enterobacteriaceae | Citrobacter | Citrobacter freundii | ATCC 8090 | 1.5 | 2.1 | 1.8 |
| Enterobacteriaceae | EKS-Enterobacter | Enterobacter aerogenes | 20/92 | 1.6 | 1.8 | 1.8 |
| Entembacteriaceae | EKS-Enterobacter | Enterobacter cloacae | 648 | 1.5 | 1.5 | 1.6 |
| Enterobacteriaceae | EKS-Enterobacter | Enterobacter sakazakii | 4306/04 | 1.5 | 7.5 | 10.8 |
| Enterobacteriaceae | EKS-Klebsiella | Klebsiella oxytoca | 275/90 | 1.4 | 2.4 | 1.6 |
| Enterobacteriaceae | EKS-Klebsiella | Klebsiella pneumoniae | 2867/81 | 1.5 | 1.8 | 1.8 |
| Enterobacteriaceae | EKS-Serratia | Serratla marcescens | 4508 | 1.6 | 1.7 | 1.7 |
| Enterobacteriaceae | Erwinia | Erwinia amylovora | E.a. 138 | 1.4 | 1.6 | 1.5 |
| Enterobacteriaceae | Escherichia | Escherichia coli | NM1 | 1.4 | 2.9 | 1.6 |
| Enterobacteriaceae | Escherichia | Escherichia coli | LMG 21756 | 1.5 | 3.4 | 1.7 |
| Enterobacteriaoeae | Hafnia | Hafnia alvei | 75-3 | 1.5 | 1.9 | 1.6 |
| Enterobacteriaceae | PMP-Morganella | Morganella morganii | NM13 | 1.6 | 3.7 | 5.4 |
| Entembacteriaceae | PMP-Proteus | Proteus vulgaris | 718/91 | 1.6 | 2.7 | 1.7 |
| Enterobacteriaceae | PMP-Providencia | Providencia stuartii | ATCC 33672 | 1.7 | 3.0 | 3.4 |
| Enterobacteriaceae | Salmonella | Salmonella enteritidis | 05/07992 | 1.5 | 1.8 | 1.8 |
| Enterobacteriaceae | Salmonella | Salmonella typhimurium | 8500/06 | 1.5 | 1.6 | 1.8 |
| Enterobacteriaceae | | Pantoea agglomerans spp.1 | 16-2 | 1.6 | 2.1 | 1.8 |
| Pseudomonadaceae | Pseudomonas | pseudomonas aeruginosa | OSMZ 50071 | 1.6 | 1.8 | 3.6 |
| Pseudomonadaceae | Pseudomonas | Pseudomonas savastanol | Ps 230 | 1.6 | 1.8 | 1.7 |
| Staphylococcaceae | Staphylococcus | Staphylococcus aureus | ATCC25923 | 1.2 | 1.3 | 1.3 |
| Staphylococcaceae | Staphylococcus | Staphylococcus haemolyticus | 06-01354 | 1.5 | 1.6 | 1.6 |

Substrate **5** yielded a distinct signal after 20 h of incubation with *E. sakazakii.* Signal to noise ratio could be further improved by prolonged incubation. Potential false positive include *Morganellea morganii* and *Providencia stuartii.*

### Example 2: Assay of Enterobacteriacae with Substrates 6 and 11.

Samples were inoculated in 200 µl nutrient broth containing Substrates **6** and **11** in 0.1 mM concentration and incubated for 22 hours at 37°C. Relative fluorescence units were determined using a standard plate reader. The amount of remaining substrate can readily be determined by the addition of sodium periodate. Results are shown in Table 4.

Substrate **6** is susceptible to *Citrobacter, Enterobacter, Hafina, Klebsiella, Escherichia, Morgenella, Proteus, Serratia (except for S. rubidea), Salmonella and Shigella spp*. but not to *Yersinia and Providencia spp*. Outside the family of *Enterobacteriacae only Bacillus* and some *Aeromonas* species gave positive results.

Substrate **11** is susceptible to *Citrobacter, Escherichia and Salmonella but is inert towards Enterobacter, Hafina, Klebsiella, Morganella, Proteus and, Shigella spp.* which can readily be assayed with Substrate **6.**

**TABLE 4: Assay of Enterobacteriacae**

| **Genus** | **Species** | **Strain** | **6** | | **11** | |
|---|---|---|---|---|---|---|
| Citrobacter | Citrobacter arnalon_aticus | 9020-77 | 56972 | + | 70629 | + |
| Citrobacter | Citrobacter braakii | 1292/04 | 50587 | + | 74038 | + |
| Citrobacter | Citrobacter diversus | 12860/88 | 97671 | + | 84083 | + |
| Citrobacter | Citrobacter freundii | ATCC 8090 | 34727 | + | 64105 | + |
| Citrobacter | Citrobacter werkmanii | 2476/04 | 52387 | + | 68751 | + |
| EKS-Enterobacter | Enterobacter aerogenes | NM 20 | 57718 | + | 2430 | - |
| EKS-Enterobacter | Enterobacter aerogenes | NM 20 | 52732 | + | 2582 | - |
| EKS-Enterobacter | Enterobacter amnigenus biovar 1 | | 40566 | + | 2216 | - |
| EKS-Enterobacter | Enterobacter cloacae | 648 | 23390 | + | 2298 | - |
| EKS-Enterobacter | Enterobacter cloacae | 648 | 19462 | + | 2145 | - |
| EKS-Enterobacter | Enterobacter sakazakii | 4306/04 | 19400 | + | 2408 | - |
| EKS-Enterobacter | Enterobacter amnigenus biovar 1 | | 40255 | + | 2574 | |
| EKS-Enterobacter | Enterobacter sakazakii | 4306/04 | 24102 | + | 2398 | - |
| EKS-Klebsiella | Klebsiella oxytoca | 275/90 | 54964 + | | 2320 | - |
| EKS-Klebsiella | Klebsiella pneumoniae | 2867/81 | 43058 | + | 2259 | - |
| EKS-Serratia | Serratia liquefaciens | 301/85 | 21044 | + | 3311 | - |
| EKS-Serratia | Serratia marcescens | 4508 | 25866 | + | 3663 | - |
| EKS-Serratia | Serratia rubidea | 130/92 | 3763 | - | 2593 | - |
| Erwinia | Erwinia amylovora | E.a. 138 | | - | | - |
| Escherichia | Escherichia coli | NM1 | 43066 | + | 52870 | + |
| Escherichia | Escherichia coli | NCTC 10418 | 88720 | + | 94097 | + |
| Escherichia | Escherichia coli | LMG 21756 | | | | |
| Escherichia | Escherichia coli 0103 | 10285/98 | 52300 | + | 59329 | + |
| Escherichia | Escherichia coli 0145 | 2217/00 | 54167 | + | 62896 | + |
| Escherichia | Escherichia coli 026 | 4469/99 | 50987 | + | 51437 | + |
| Escherichia | Escherichia coli 091 | 4930/98 | 45613 | + | 52332 | + |
| Escherichia | Escherichia coli | NM1 | 33731 | + | 44202 | + |
| Hafnia | Hafnia alvei | 75-3 | 20387 | + | 2310 | - |
| PMP-Morganella | Morganella morganii | NM13 | 32895 | + | 5906 | - |
| PMP-Proteus | Proteus mirabilis | 671 | 34406 | + | 4708 | - |
| PMP-Proteus | Proteus vulgaris | 718/9 | 25287 | + | 3745 | - |
| PMP-Providencia | Providencia rettgeri | DSMZ 4542 | 3084 | - | 2239 | - |
| PMP-Providencia | Providencia stuartii | ATCC 33672 | 3552 | - | 2863 | - |
| Salmonella | Salmonella agona | 3515/03 | 27163 | + | 81556 | + |
| Salmonella | Salmonella agona | 4005/03 | 26759 | + | 80382 | + |
| Salmonella | Salmonella bongori | RKI 1709 | 42894 | + | 92304 | + |
| Salmonella | Salmonella bongori | RKI 1300 | 61961 | + | 98930 | + |
| Salmonella | Salmonella enteritidis | 05/07992 | 25467 | + | 70613 | + |
| Salmonella | Salmonella enteritidis | 05/07992 | 18845 | + | 57473 | + |
| Salmonella | Salmonella enteritidis | 05/07992 | 29218 | + | 83729 | + |
| Salmonella | Salmonella enteritidis | 8509/06 | 25907 | + | 81284 | + |
| Salmonella | Salmonella enteritidis | 1994/06 | 24579 | + | 79593 | + |
| Salmonella | Salmonella gallinarum | 527 | 3474 | - | 19735 | + |
| Salmonella | Salmonella II | 6039319 | 32027 | + | 82876 | + |
| Salmonella | Salmonella II | 1150/96 | 39339 | + | 86408 | + |
| Salmonella | Salmonella IIIa | 3274/81 | 36407 | + | 86575 | + |
| Salmonella | Salmonella IIIa | RKI 1612 | 44363 | + | 71444 | + |
| Salmonella | Salmonella IIIb | ES 43/02 | 37674 | + | 88515 | + |
| Salmonella | Salmonella IIIb | 44/02 2634 | 37668 | + | 2634 | o |
| Salmonella | Salmonella pullorum | S2 1909/93 | 30009 | + | 58895 | + |
| Salmonella | Salmonella pullorum | S2 1908/93 | 34612 | + | 71353 | + |
| Salmonella | Salmonella typhimurium | 8500/06 | 25379 | + | 80669 | + |
| Salmonella | Salmonella typhimurium | 8541/06 | 25638 | + | 79064 | + |
| Salmonella | Salmonella VI | RKI 1609 | 45316 | + | 86509 | + |
| Salmonella | Salmonella VI | RKI 864 | 40902 | + | 100955 | + |
| Shigella | Shigella boydii | 03/7455 | 56963 | + | 54845 | + |
| Shigella | Shigella dysenteriae | 00/2085 | 32666 | + | 66466 | + |
| Shigella | Shigella flexneri | 03/3709 | 79688 + | | 75266 | + |
| Shigella | Shigella sonnei 02/3828 | 02/3828 | 40728 | + | 63820 | + |
| Yersinia | Yersinia enterocolitica 06/3968 | 06/3968 | 3423 | - | 2167 | - |
| | Pantoea agglomerans spp.1 16-2 | | 25719 | + | 2274 | - |

| | | | | | | |
|---|---|---|---|---|---|---|
| Legend o Low optical density due to poor growth - Negative Result: RFU < 10'000 + Positive Result: RFU > 20'000 RFU Relative Fulorescence Units | | | | | | |

### Example 3: Assay of Salmonella with Substrate 15

Samples were inoculated in 200 µl nutrient broth containing Substrate **15** in 0.1 mM concentration and incubated for 22 hours at 37°C. Relative fluorescence units were determined after 10h 15h and 20h using a standard plate reader. Results are shown in Table 5.

**TABLE 5: Assay of Salmonella**

| **Genus** | **Species** | **Strain** | **10 h** | **15h** | **20 h** |
|---|---|---|---|---|---|
| | | | | | |
| | control | | 2967 | 3059 | 3045 |
| Staphylococcus | Staphylococcus aureus | ATCC 25923 | 3078 | 3155 | 3220 |
| Escherichia | Escherichia coli | NCTC 10418 | 2968 | 3088 | 3072 |
| Salmonella | Salmonella II | 6039319 | 2940 | 2856 | 5791 |
| Salmonella | Salmonella II | 1150/96 | 2900 | 2917 | 6349 |
| Salmonella | Salmonella IIIa | 3274/81 | 3180 | 3869 | 10963 |
| Salmonella | Salmonella IIIa | RKI 1612 | 2936 | 3007 | 3152 |
| Salmonella | Salmonella IIIb | ES 43/02 | 2800 | 3192 | 5977 |
| Salmonella | Salmonella IIIb | ES 44/02 | 2805 | 3279 | 6035 |
| Salmonella | Salmonella VI | RKI 1609 2834 | | 3037 | 3340 |
| Salmonella | Salmonella VI | RKI 864 | 2870 | 2934 | 3516 |
| Salmonella | Salmonella agona | 3515/03 | 3147 | 4085 | 9635 |
| Salmonella | Salmonella agona | 4005/03 | 3691 | 4312 | 9485 |
| Salmonella | Salmonella bongori | RKI 1709 | 3062 | 3189 | 8553 |
| Salmonella | Salmonella bongori | RKI 1300 | 3003 | 4445 | 21643 |
| Salmonella | Salmonella enteritidis | RKI 05/07992 | 3246 | 4889 | 15245 |
| Salmonella | Salmonella enteriditis | 8509106 | 3054 | 3933 | 12614 |
| Salmonella | Salmonella enteriditis | 1994/06 | 3138 | 3923 | 12603 |
| Salmonella | Salmonella gallinarum | 527 | 2834 | 3037 | 3340 |
| Salmonella | Salmonella pullorum | S2 1909/93 | 2869 | 2937 | 4370 |
| Salmonella | Salmonella pullorum | S2 1908/93 | 2780 | 3197 | 4115 |
| Salmonella | Salmonella typhimurium | 8541/06 | 3062 | 3441 | 8749 |
| Salmonella | Salmonella typhimurium | 8500/06 | 3075 | 3773 | 9346 |

| | | | | | |
|---|---|---|---|---|---|
| *) Strains "RKI 1612" and "527" grew weakly (OD600 below 0.3 while 0.6 was expected) | | | | | |

### Example 4: Assay of Enterobacteriacae with Substrate 16.

Samples were inoculated on nutrient agar plates (5g/l peptone, 5g/l NaCl, 2 g/l yeast extract, 1 g/l meat extract, 14 g/l agar, pH 7.4) containing 0.3 mM and 0.9 mM of Substrate **16** and incubated at 37°C for 30 hours. Growth and color of various species are shown in Table 6.

**TABLE 6: Microbial Plating Medium**

| **Species** | **Strain** | **0.3 mM** | **0.9 mM** |
|---|---|---|---|
| Escherichia coli | NM1 | Good growth, olive-green | Good growth, olive-green (darker than at 0.3 mM) |
| Salmonella enteritidis | 05/07992 | Good growth, colorless | Good growth, light yellow-brown coloration |
| Staphylococcus aureus | ATCC 25923 | Pinpoint | No growth |
| Bacillus subtilis | ATCC 6633 | Weak growth, colorless | No growth |

In the experiments performed, Substrate **16** specifically stained species of the family of *Enterobacteriacae*. Interestingly, Substrate **16** seemed to inhibit the growth of gram positive species, thereby enhancing specificity.

### Example 5: Synthesis of Substrate 15 starting from Substrate 11

### rac-7-(4'-Boc-L-alaninamido-3'-hvdroxybutyloxy-2H-1-benzopyren-2-one (Compound 21)

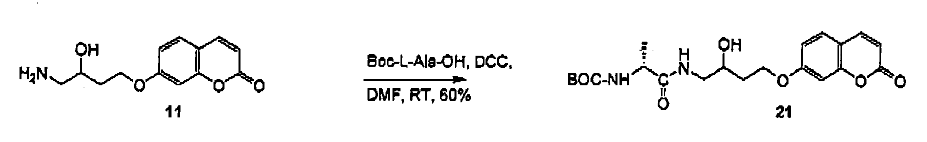

Under nitrogen atmosphere a solution of 3.74 g (15 mmol; M=249,2 g/mol) rac-7-(4'-amino-3'-hydroxybutoxy)-2H-1-benzopyran-2-one (Substrate **11**) and 5.68 g (30 mmol; M=189.4 g/mol) Boc-L-Alanine in 70 ml DMF was treated under stirring with 3.57 g (17.5 mmol; M=206.3 g/mol) DCC. The mixture was stirred for 5 hours at room temperature. Then the suspension was filtered, the DCC-urea washed with 10 ml DMF and the combined filtrates diluted with 200 ml Na₂CO₃-saturated solution. The reaction product was extracted with three 50ml portions of ethyl acetate. The ethyl acetate fractions where combined, washed with brine and evaporated in vacuum. The residue was purified by crystallization (acetone/diethyl ethe/petroleum ether (40/60)) to yield 3.8 g (60%; M=420.6 g/mol) of Compound **21.**

Product analysis: TLC [petroleum ether (40/60)/ ethyl acetate; (1:2)]: R_{F}=0.1. ¹H-NMR [DMSO-d6]: 1.15 (d, 3H, J= 7.0 Hz), 1.35 (s, 9H), 1.65-1.73 (m, 1H), 1.83-1.92 (m, 1H), 3.03- 3.20 (m, 2H), 3.65-3.75 (m, 1H), 3.89-3.99 (m, 1H), 4.15 (t, 2H, J= 6.3 Hz), 4.85 (d, 1H, OH, J=5.1 Hz), 6.25 (d, 1H, J= 9.5 Hz), 6.84 (bd, 1H, NH), 6.89-6.96 (m, 2H), 7.59 (d, 1H, J= 8.5 Hz), 7.69 (bt, 1H, NH), 7.96 (d, 1H, J= 9.5 Hz).

### rac-7-(4'-Boc-L-Alaninamido-3'-caproylonbulyloxv)-2H-1-benzopyran-2-one (Compound 22)

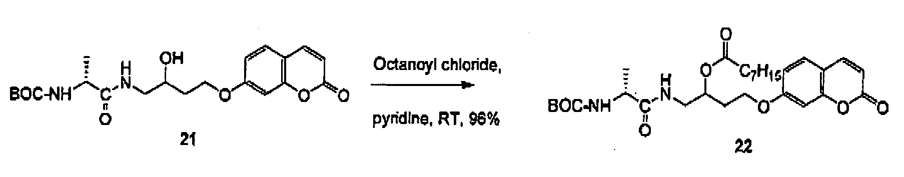

Under nitrogen atmosphere 3.43 g (21 mmol, M=162.7 g/mol) octanoyl chloride was added to a stirred solution of 6 g (14 mmol; M=240.6 g/mol) Compound **21** in 25 ml pyridine at room temperature. The mixture was stirred for 4 hours at room temperature. The resulting suspension was evaporated in vacuum and the residue was dissolved in 200 ml dichloromethane/water (1:1). Concentration and chromatography (petroleum ether/ ethyl acetate, (2:1) to (1.75:1)) of the organic layer gave 7.4 g (96%; M=546.8) Compound **22** as a syrup.
Product analysis: TLC [petroleum ether (40/60)/ ethyl acetate; (2:1)]: R_{F}=0.3. ¹H-NMR [DMSO-d6]: 0.79 (t, 3H, J= 6.1 Hz), 1.09-1.22 (m, 11H), 1.36 (s, 9H), 1.44-1.48 (m, 2H), 1.88-2.07 (m, 2H), 2.22-2.28 (m, 2H), 3.13- 3.46 (m, 2H), 3.87-4.16 (m, 3H), 4.98-5.07 (m, 1H), 6.28 (d, 1H, J= 9.5 Hz), 6.86-6.96 (m, 3H, NH), 7.61 (d, 1H, J= 8.6 Hz), 7.83-7.95 (m, 1H, NH), 7.99 (d, 1H, J= 9.0 Hz).

### rac-7-(4'-L-Alaninamido-3'-caproyloxybuty)-2H-1-benzopyran-2-one, TFA salt (Substrate 15)

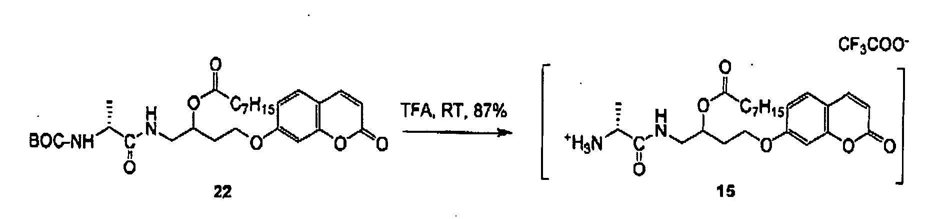

Under nitrogen atmosphere 7.4 g (13.5 mmol; M=546.8 g/mol) of the Boc-protected Compound **22** and 25 ml trifluoroacetic acid were stirred at room temperature for 1 hour. Thereby, Compound **22** gradually dissolves. Evaporation in vacuum and chromatography (ethyl acetate/ methanol, (15:1) to (10:1)) of the residue yielded 4.9 g (87%; M=560.6) of Substrate **15** as a hygroscopic foam.
Product analysis: TLC [ethyl acetate/ methanol; (10:1)]: R_{F}=0.1. ¹H-NMR [DMSO-d6]: 0.77 (t, 3H, J= 6.8 Hz), 1.03-1.20 (m, 8H), 1.30-1.34 (m, 3H), 1.42-1.46 (m, 2H), 1.91-2.08 (m, 2H), 2.24-2.28 (m, 2H), 3.15- 3.58 (m, 2H), 3.84-3.87 (m, 1H) 4.04-4.15 (m, 2H), 5.04-5.09 (m, 1H), 6.26 (d, 1H, J= 9.5 Hz), 6.88-6.94 (m, 2H), 7.59 (d, 1H, J= 8.6 Hz), 7.97 (d, 1H, J= 9.5 Hz), 8.25 (s, 3H, NH₃), 8.63-8.71 (m, 1H, NH).

### Example 6: Synthesis of Substrate 16

### rac-5-Bromo-4-chloro-3-(3',4'-O-isopropylidene-3',4'-dihydroxybutyloxy)-indoxyl-1-acetate (Compound 24)

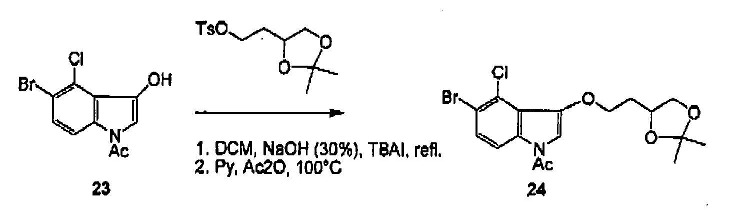

Under nitrogen atmosphere 82.5 g (275 mmol; M=300 g/mol) 1,2-O-isopropylidene-butane-1,2,4-triol 4-p-toluenesulfonate and 53.14 g (185 mmol; M=289 g/mol) 5-bromo-4-chloro-3-indoxyl-1-acetate (Compound **23**) were added under stirring to a biphasic mixture of 13.7 g (37 mmol, M=369) TBAI in 367 ml 30% NaOH and 367 ml dichloromethane. The mixture was vigorously stirred for 48 hours at 45°C. Then the organic phase was separated, washed with 500 ml water and evaporated in vacuum/40°C. The resulting syrup was co-evaporated with 500 ml toluene/methanol (10:1) and acetylated in 250 ml pyridine and 250 ml acetic anhydride at 100°C over night.
Concentration in vacuum/60°C and chromatography of the oily residue (silica gel; petroleum ether/ ethyl acetate, (4:1)) gave 35 g of crude Compound **24** as slowly crystallizing syrup.
Purification of the crude product was achieved by acetone extraction, concentration of the acetone extract and crystallization at 0°C. Yield: 28g (36%) pure Compound **24** as an off-white powder.
Product analysis: TLC [petroleum ether (40/60)/ ethyl acetate; (4:1)]: R_{F}=0.2. ¹H-NMR [CDCl₃]: 1.37 (s, 3H), 1.44 (s, 3H), 2.10-2.17 (m, 2H), 2.55 (s, 3H), 3.71 (dd, 1H, J = 7.0 Hz, J = 8.0 Hz), 4.09-4.12 (m, 2H), 4.17 (dd, 1H, J = 6.0 Hz, J = 8.2 Hz), 4.34-4.41 (m, 1H), 6.81 (s, 1H), 7.52 (d, 1H, J= 8.9 Hz), 8.22 (d, 1H, J= 8.7 Hz).

### rac-5-Bromo-4-chloro-3-(3',4'-dihydroxybutyloxy)-indoxyl (Substrate 16)

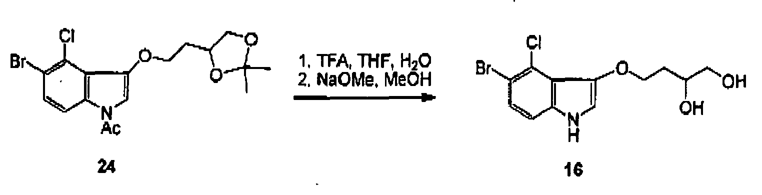

Under nitrogen atmosphere 6.0 g (14.3 mmol, M=417 g/mol) Compound **24** was stirred in a mixture of 60 ml tetrahydrofuran, 20 ml water and 5 ml trifluoroacetic acid at room temperature until TLC [[toluene/acetone, (1:1)]: R_{F}=0,15] indicated complete conversion of the starting material.
Filtration, concentration of the filtrate in vacuum/60°C and chromatography of the oily residue (silica, gel; toluene/ acetone (1.1) to (1:1.5)) yielded 2 g of foamy isopropylidene-de-protected intermediate, which was de-acetylated under Zemplen-conditions in a mixture of 0.5 ml methanolic NaOMe-solution (5.4 M) and 15 ml methanol at RT. After neutralization with Dowex MS650 (H⁺) the mixture was evaporated in vacuum/40°C.
Chromatography (silica gel; toluene/acetone, (1:1)) of the residue yielded 2 g of crude Substrate **16** as slowly crystallizing syrup. Crystallization from MTBE yielded 1.5 g (27%) pure Substrate **16** as a white powder.
Product analysis: TLC [MTBE]: R_{F}=0.1. ¹H-NMR [DMSO-d6]: 1.61-1.71 (m, 1H), 1.92-2.01 (m, 1H), 3.32-3.39 (m, 2H), 3.66-3.74 (m, 1H), 4.00-4.04 (m, 2H), 4.53-4.57 (m, 2H, 2 OH), 7.09 (bs, 1H), 7.19 (d, 1H, J= 8.7 Hz), 7.29 (d, 1H, J= 8.7 Hz), 10.97 (bs, 1H, NH).

### Example 7: Synthesis of Substrate 17

### Sodium 4-chloro-2-(6-chloro-4-oxo-3,4-dihydroquinazoline-2-yl)phenolate

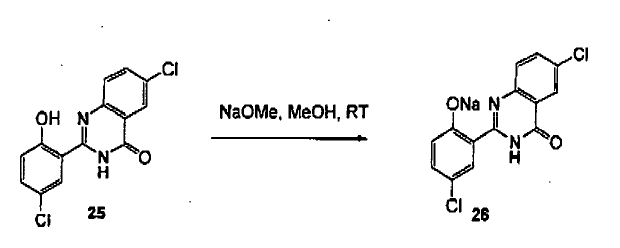

Under nitrogen atmosphere a suspension of 0.5 g (1.63 mmol; M=306.9 g/mol) 6-Chloro-2-[5-chloro-2-hydroxyphenyl]-quinazoline₋4(3H)-one (Compound **25**) in 25 ml absolute methanol was treated with 0.3 ml (1.62 mmol) of a 5.4M methanolic NaOMe-solution at room temperature for 15 minutes. Evaporation of the reaction mixture in vacuum/60° and drying in vacuum over night yielded the sodium salt 26 as a yellow solid.

### 6-chloro-2-(5-chloro-2-(2-(2,2-dimethyl-1,3-dioxolane-4-yl)ethoxy)phenyl)-quinazoline-4(3H)-one (Compound 27)

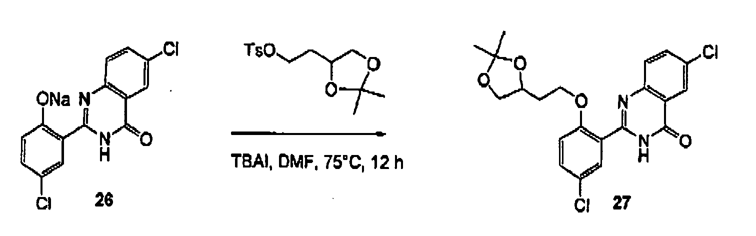

Compound **26** was dissolved under nitrogen atmosphere in 25 ml DMF at room temperature and 1 g (3.3 mmol; M=300.4 g/mol) 1,2-O-Isopropyliden-butane-1,2,4-triol 4-p-toluenesuffonate and 0.1 g (0.27 mmol, M=369.4) TBAI were added under stirring. The mixture was vigorously stirred for 12 hours at 75°C. Then 50 ml ethyl acetate, 50 ml water and 50 ml brine were added and stirring was continued for 15 minutes before the organic layer was separated. Evaporation of the organic phase in vacuum/40°C and chromatography (silica gel; petroleum ether (40/60)/ ethyl acetate, (5:1) to (3:1)) gave 0.43 g (61%, M=434.9 g/mol) of Compound **27** as a solid. TLC [petroleum ether (40/60)/ ethyl acetate (4:1)]: RF=0.15.

### rac-6-Chloro-2-[5'-chloro-2'-(3",4"-dihdroxybutyloxy)phenyl]-quinazoline-4(3H)-one (Substrate 17)

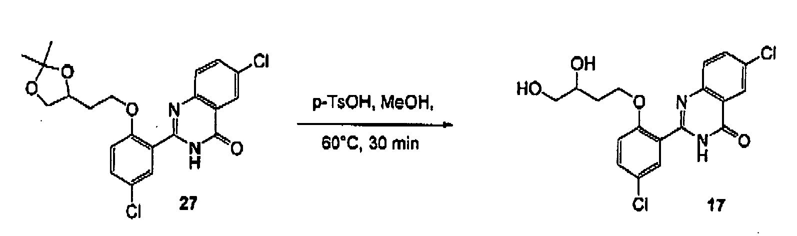

De-protection of Compound **27** was achieved by heating 0.43 g of the isopropylidene protected coupling product in 50 ml acidulated (0.2 g p-TsOH) methanol. Evaporation of the resulting solution in vacuum/60°C and purification by crystallization (2-propanol) yielded 0.3 g (76 %, M= 394.9 g/mo!) Substrate **17** as a colorless solid. Product analysis: TLC [petroleum ether (40/60)/ ethyl acetate (1:1)]: RF=0.1. 1H-NMR [DMSO-d6/ D20]: 1.63-1.72 (m, 1H), 1.86-1.94 (m, 1H), 3.23-3.34 (m, 2H), 3.56-3.62 (m, 1H), 4.18 (t, 2H, J = 6.5 Hz), 7.22 (d, 1H, J = 9.0 Hz), 7.56 (dd, 1H, J = 2.8 Hz, J = 8.9 Hz), 7.73-7.76 (m, 2H), 7.86 (dd, 1H, J = 2.5 Hz, J = 8.7 Hz), 8.07 (d, 1H, J = 2.5 Hz),

### Example 8: Synthesis of Substrate 18

### rac-10-Acetyl-3,7-di-O-(3',4'-O-isopropylidene-3',4'-dihydroxybutyloxy)-phenoxazine (Compound 29)

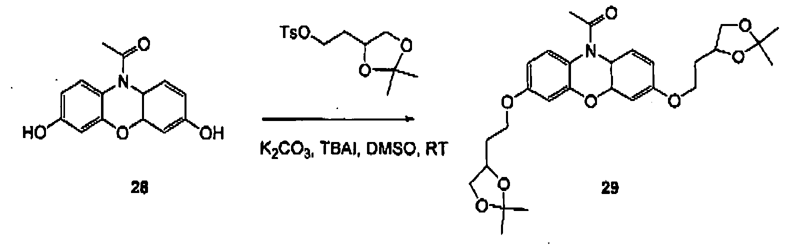

Under nitrogen atmosphere 35 g (116.5 mmol; M=300.4 g/mol) 1,2-O-isopropylidene-butane-1,2,4-triol 4-p-toluenesuffonate and 10 g (38.6 mmol; M=259:3 g/mol) 10-acetyl-3,7-dihydroxyphenoxazine (Compound **28**, synthesized according to patent DE 3644401) were added under stirring to a suspension of 0.75 g (2 mmol, M=369.4) TBAI and 12.5 g (90.4 mmol, M=138.2 g/mol) anhydrous potassium carbonate in 25 ml dimethyl sulfoxide. The mixture was vigorously stirred for 5 days at 25°C. Then 100 ml ethyl acetate and 100 ml water were added and the organic phase was separated. The aqueous phase was extracted twice each with 100 ml ethyl acetate. The combined ethyl acetate phases were evaporated in vacuum/40°C and subsequent chromatography of the oily residue (silica gel; petroleum ether/ ethyl acetate, (4:1) to (1.5:1)) gave 17.3 g (87%, M=515.6) of Compound **29** as a yellow syrup.
Product analysis: TLC [toluene/ ethyl acetate; (1:1)]: RF=0.5. 1H-NMR [DMSO-d6]: 1.25 (s, 6H), 1.31 (s, 6H), 1.89-1.96 (m, 4H), 2.19 (s, 3H), 3.55 (t, 2H, J = 7.9 Hz), 4.00-4,18 (m, 6H), 4.15-4.20 (m, 2H), 6.71-6.77 (m, 4H), 7.45 (d, 2H, J= 8.7 Hz).

### rac-10-Acetyl-3,7-di-O-(3',4'-dihydroxybutyloxy)-phenoxazine (Substrate 18)

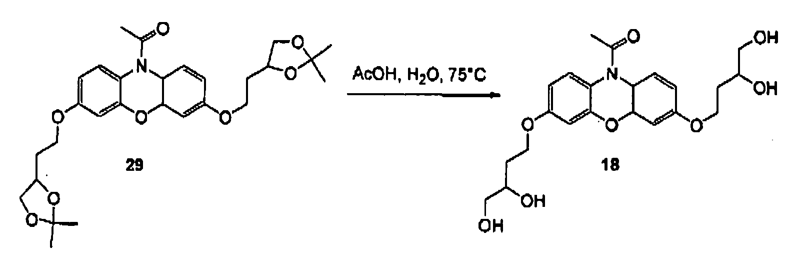

Under nitrogen atmosphere 2.23 g (4.34 mmol, M=515.6 g/mol) of Compound 29 was stirred in a mixture of 25 ml acetic acid and 25 ml water at 75°C for 2 hours. Concentration of the reaction mixture in vacuum/60°C and chromatography of the oily residue (silica gel; EE to EE/MeOH (10:1)) yielded 0.6 g of Compound **18** as orange-colored foam. Decolorization with charcoal in methanol/ ethyl acetate (10:1) gave after filtration and evaporation in vacuum 0.55g (27%) of Compound **18** as nearly colorless foam.

Product analysis: TLC [EE/ MeOH (10:1)]: RF=0.25. 1H-NMR [DMSO-d6]: 1.57-1.66 (m, 2H), 1.86-1.94 (m, 2H), 2.19 (s, 3H), 3.25-3.44 (m, 4H), 3.57-3.65 (m, 2H), 4.05-4.09 (m, 4H), 4.54 (t, 2H, J = 5.7 Hz, OH), 4.61 (d, 2H, J = 5**.**1 Hz, OH), 6.71-6.75 (m, 4H), 7.45 (d, 2H, J= 8.7 Hz).

### Example 9: Synthesis of Substrate 13

### 5-Oxotetrahvdrofuran-2-carbonlic acid (31)

This compound was prepared starting from DL-glutamic acid **(30)** according to the procedure of Rouessac et al., Organic Syntheses, Coll. Vol,7, p. 99 (1990).

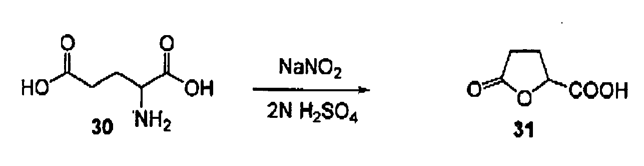

### 1,2-O-Isopropylidene-1,2,5-pentantriol (32)

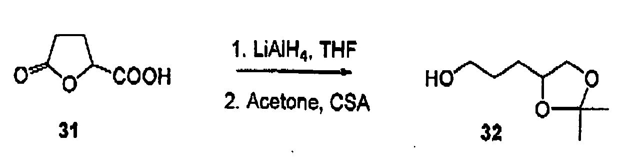

Under nitrogen atmosphere a solution of 24 g (184.5 mmol; 130.1 g/niol) of **31** in 150 ml dry tetrahydrofuran was slowly (within 1 hour) dropped into a stirred suspension of 9.5 g (250,3 mmol; M=37.95 g/mol) LiAlH₄ in 300 ml dry tetrahydrofuran at 40-50°C. The suspension was stirred for 4 hours at 50°C.

After cooling, excess LiAlH₄ was destroyed by slowly adding 5 ml water (caution: drop by drop!) and 1N sulphuric acid (caution!) until phase separation occurred (ca. 150 ml) under vigorous stirring.
The organic phase was separated and the aqueous phase was extracted twice each with 100 ml tetrahydrofuran. The combined tetrahydrofuran phases were evaporated in vacuum/40°C and subsequent chromatography of the oily residue (silica gel; ethyl acetate/ methanol, (8:1) to (6:1)) gave 16.9 g (76%, M=120.2 g/mol) of the intermediate1,2,5-pentantriol as a yellow syrup. TLC [dichloromethane/ methanol; (9:1)]: RF=0.17.
8 g (66.6 mmol; M=120.2 g/mol) 1,2,5-Pentantrio) was stirred in 80 ml acetone with 200 mg of (+/-)-camphor-10-sulfonic acid at room temperature for 24 hours. After neutralization of the solution with triethylamine the acetone was evaporated in vacuum/40°C. Chromatography of the oily residue [silica gel; petroleum ether (40/60)/ethyl acetate, (4:1) to (2:1)] gave 9.2 g (86%. M=160.2 g/mol) of Compound **32** as a colorless oil.
Product analysis: TLC [petroleum ether (40/60)/ ethyl acetate; (2:1)]: RF=0.31. 1H-NMR [DMSO-d6]: 1.24 (s, 3H), 1.29 (s, 3H), 1.38-1.54 (m, 4H), 3.38-3.42 (m, 3H), 3.95-4.03 (m, 2H), 4.38 (t, 1H, OH, J=5.2 Hz).

### 1,2-O-Isopropylidene-pentane-1,2,5-triol 4-p-toluenesulfonate (Compound 33)

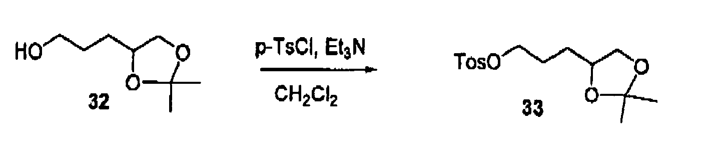

Under nitrogen atmosphere a solution of 6.6 g (41.2 mmol; M=160.2 g/mol) of Compound **32** in 37 ml dry dichloromethane and 8.7 ml (62.4 mmol) triethylamine was cooled under stirring to 5°C. 9.4 g (49.3 mmol; 190.65 g/mol) p-toluenesulfonyl chloride was added dropwise as a solution in 9 ml dry dichloromethane. The mixture was stirred for 1 hour at 5°C and then allowed to warm to room temperature over night. Then 50 ml water was added and the organic phase was separated, dried over MgSO4 and concentrated in vacuum/40°C. Chromatography of the oily residue (silica gel; petroleum ether/ ethyl acetate, (6:1)) gave 11.6 g (89%, M=314.4 g/mol) of Compound **33** as a slightly yellow syrup.
Product analysis: TLC [petroleum ether (40/60)/ ethyl acetate; (6:1)]: RF=0.38.

### rac-7-(4',5'-Dihydroxypentyloxy)-2H-1-benzopyran-2-one (Substrate 13)

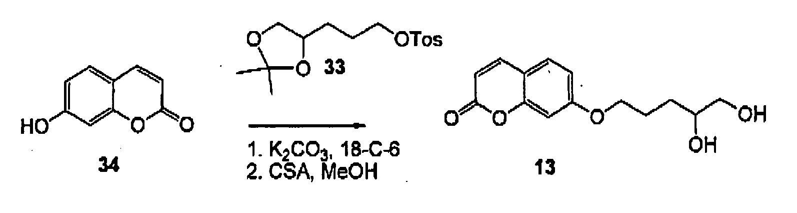

Under nitrogen atmosphere a suspension of 11.55 g (36.7 mmol; M=314.1 g/mol) 1,2-O-Isopropylidene-pentane-1,2,5-triol 4-p-toluenesulfonate, 5.87 g (36.2 mmol; M=162.15 g/mol) of Compound **35**, 10.02 g anhydrous potassium carbonate and 1.91 g (7.23 mmol; M=264.32 g/mol) 18-crown-6 in 140 ml dry acetone was refluxed under vigorous stirring for 6 hours.
After evaporation of the mixture in vacuum/40°C 130 ml ethyl acetate and 130 ml 1 N sodium hydroxide solution were added and the organic phase was separated. The aqueous phase was extracted with 100 ml ethyl acetate and the combined ethyl acetate phases were evaporated in vacuum/40°C to yield 10 g of the coupling product as an oily residue which was deprotected without further purification. TLC [petroleum ether/ ethyl acetate (4:1)]: RF=0.28.

10 g of the crude coupling product was stirred in 200 ml of acidified (1.5 g (+/-)-camphor-10-sulfonic acid) methanol for 5 hour at room temperature. After evaporation of the reaction mixture in vacuum/40°C 40 ml 0.5N NaOH was added. The product was extracted with ethyl acetate (4 x 75 ml). Evaporation of the combined ethyl acetate fractions and crystallization from methyl ethyl ketone at 0°C gave 5 g of Compound **13** (52 %, M=264.28 g/mol) as a white solid. Further MEK re-crystallization yielded analytical pure material.
Product analysis: TLC [dichloromethane/ methanol (20:1)]: RF=0.3. 1H-NMR [DMSO-d6]: 1.30-1.40 (m, 1H), 1.56-1.91 (m, 3H), 3.23-3.46 (m, 3H), 4.06-4.09 (m, 2H), 4.53 (bs, 2H, 20H), 6.27 (d, 1H, J=9.5 Hz), 6.91-6.96 (m, 2H), 7.60. (d, 1H, J= 8.6 Hz), 7.97 (d, 1H, 9.5 Hz).

### Example 10: Synthesis of Substrate 20

### 2-(2,2-Dimethyl-1,3-dioxolan-4-yl)ethyl 1H-indol-3-ylcarbamate (Compound 36)

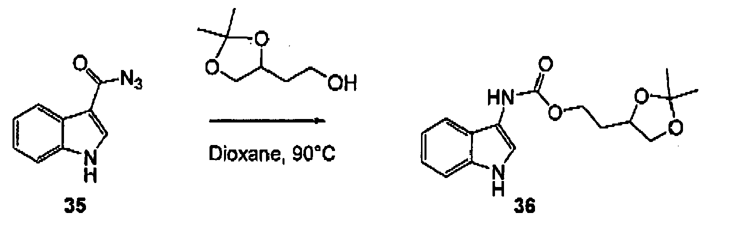

Indole-3-carboxylic acid azide (3.0 g, 16.11 mmol, prepared according to Slouka et al. Collection Czechoslov. Chem. Commun. 43 (1978), 960-965), was dissolved in 120 ml dry dioxane and heated to 90°C for 45 minutes. 2-(2,2-dimethyl-1,3-dioxolan-4-yl)ethanol (7.07 g;48.36mmol) was added and stirred at 90°C for further 6 hours. The reaction mixture was filtered while still hot, the filtrate evaporated to dryness resulting in 9.22 g brown oil. The crude product was purified by column chromatography (SiO₂; toluene) yielding 2.57 of Compound **36** (52.4%) as a brownish oil.
Product analysis: 1H-NMR(CDCl3): 8.17 (bs, 1H), 7.50-7.48 (d, 1H), 7.42 (b, 1H), 7.32-7.30 (d, 1H), 7.24-7.10 (m, 3H), 4.40-4.34 (m, 1H); 4.30-4.24 (m, 2H), 4.13-4.09 (m, 1H), 3.64-3.57 (m, 1H). 1.97 (b, 2H), 1.44 (s, 3H), 1.38 (s, 3H).
13C-NMR(CDCl3): 154.29, 134.14, 128.18, 122.49, 119.40, 116.63, 115.61, 114.60, 111.43, 73.14, 69.29, 62.26, 60.51, 33.22, 26.93, 25,66.

### rao-3,4-Dihydroxybutyl 1H-indole-3-ylcarbamate (Substrate 20)

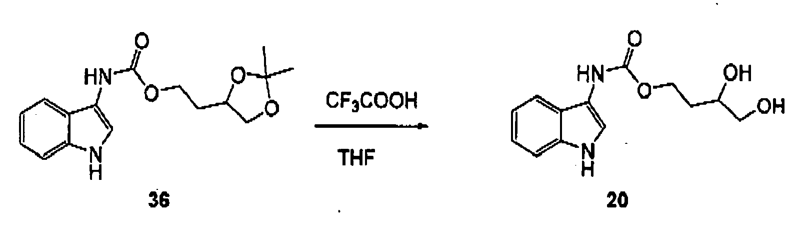

0.75g (2.46 mmol; M=304.35 g/mol) of Compound **36** was dissolved in 8 ml tetrahydrofuran. Under agitation 4 ml water and 1 ml trifluoracetic acid were added. The solution was stirred at room temperature for 1 hour.
Evaporation in vacuum/50°C and chromatography (ethyl acetate) gave 0.39 g (60%, M=264.28 g/mol) of Substrate **20** as a solidifying oil.
Product analysis: TLC (ethyl acetate): RF=0.29. 1H-NMR [DMSO-d6]: 1.51-1.59 (m, 1H), 1.80-1.89 (m, 1H), 3.23-3.68 (m, 3H), 4.16-4.22 (m, 2H), 3.3-4.6 (broad signal, 2H, 20H), 6.93 (dd, 1H, J=7.6 Hz), 7.05 (dd, 1H, J=8.0 Hz), 7.29 (d, 1H, J= 8,1 Hz), 7.40 (s, 1H), 7.71 (d, 1H, 7.6 Hz), 9.31 (s, 1H, NH), 10.71 (s, 1H, NH).

## Claims

1. A method of detecting the presence of selected living cells in a sample, comprising the steps of:
a) contacting the sample with a compound having the formula: 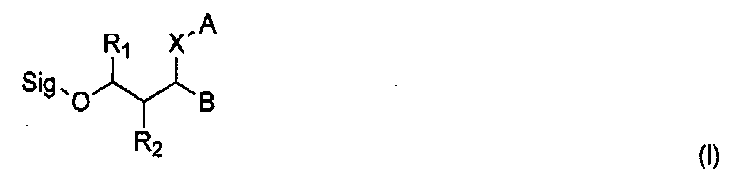 wherein:
X is O or NH,
A is H or a first enzymatically hydrolyzable group,
B is R₃ or CH-Y-D, wherein Y is O or NH and D is H or a second enzymatically hydrolyzable group,
R₁, R₂ and R₃ are independently selected inert groups, and
Sig-O is a signalogenic moiety that upon release thereof converts to a signalophore, and
b) monitoring for a signal from said signalophore caused by a metabolic activity of said selected living cells, said activity resulting in a release of said signalogenic moiety.

2. R₁, R₂ and R₃ are independently selected from H, CH₃, linear and branched lower alkyl with up to 7 carbon atoms, CI, linear and branched lower alkoxy with up to 7 carbon atoms.

3. The method according to claim 1 or 2, wherein the signalophore belongs to the families of azo-dyes, acridanes, benzenes, benzimidazoles, benzothiazoles, benzoxazoles, bipyridyls, catechols, coumarines, dioxetanes, flavones, luciferins, napthols, nitrobenzenes, indoles, indolinones, quinazolines, quinolines, phenoxazines or triphenylmethane dyes.

4. The method according to claim 1 or 2, wherein Sig is an aryloxycarbonyl or arylaminocarbonyl residue.

5. The method according to one of claims 1 to 4, wherein said first and/or said second enzymatically hydrolyzable groups are independently selected from a group that is labile towards the action of glycosidases, peptidases, ureidases, esterases, lipases, phosphatases, sulfatases, phospholipases A, B and C, dealkylases, or nitroreductases.

6. The method according to claim 1, wherein the Compound (I) is selected from the group.consisting of:

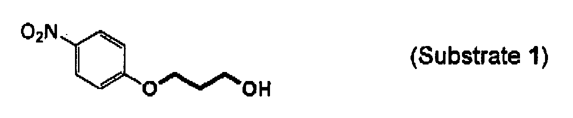

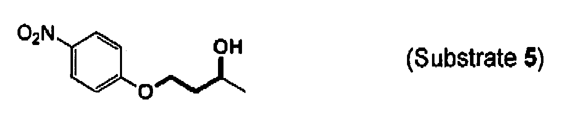

7. The method according to claim 1, wherein the Compound (1) is selected from the group consisting of:

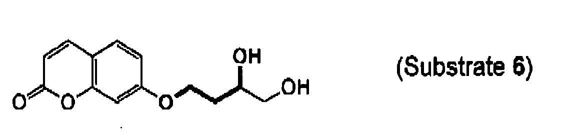

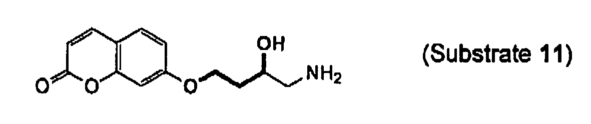

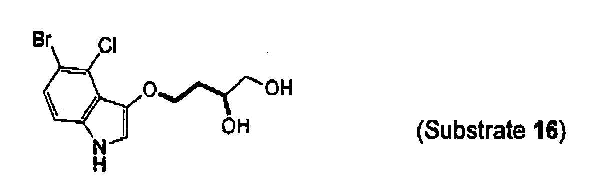

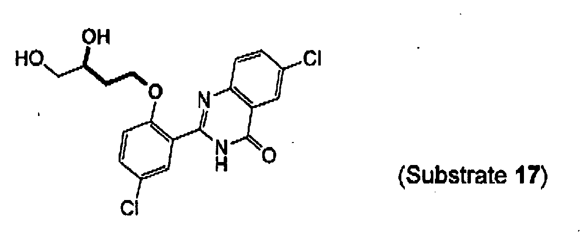

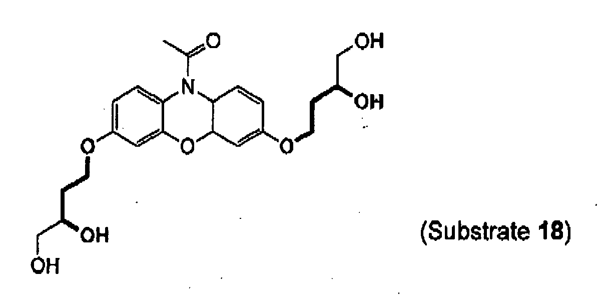

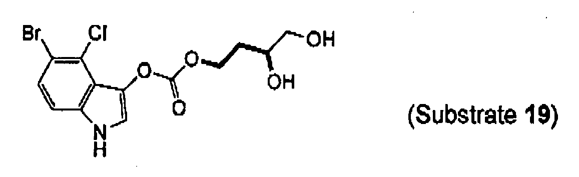

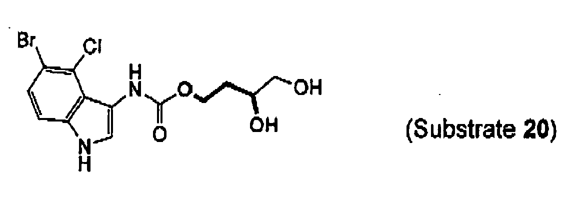

8. The method according to claim 6 or 7, wherein the living cells are from species or strains belonging to the family of *Enterobacteriaceae.*

9. The method according to claim 6, wherein the living cells are from *E. sakazakii* species.

10. The method according to claim 1, wherein A is a first enzymatically hydrolyzable group and B is a second enzymatically hydrolyzable group that is different therefrom, and wherein the living cells have enzymatic cleavage activity towards both enzyme labile groups.

11. The method according to claim 10, wherein the Compound (I) is

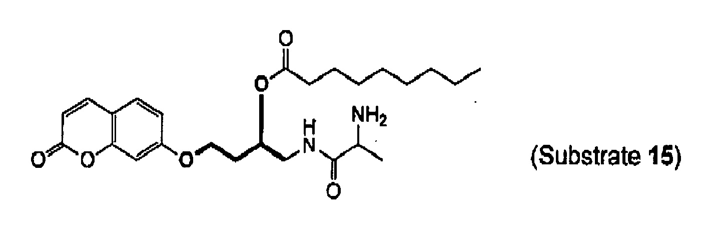

12. The method according to claim 11, wherein the living cells are from *E*. *Salmonella* species.

13. The method according to one of claims 1 to 12, wherein said contacting step comprises inoculation of a microbial sample into a culture medium containing at least one of said Compounds (I) and wherein release of said signalogenic species is effected by incubation of said inoculated culture medium.

14. The method according to claim 13, wherein said culture medium is an enrichment broth.

15. The method according to one of claims 1 to 14, wherein said signal comprises a change in optical properties.
